# EUROPEAN PATENT APPLICATION

(11) **EP 4 424 825 A1**
(43) Date of publication of application: **04.09.2024**
(21) Application number: 22887584.5
(22) Date of filing: 26.10.2022
(51) Int. Cl.: C12N 15/10, C12Q 1/6844

(54) **PRECIPITANT COMPOSITION FOR DNA EXTRACTION AND DNA EXTRACTION METHOD USING SAME**

(30) Priority: 28.10.2021 KR 20210145494
(71) Applicant: Fore Front Test, Seongnam-si, Gyeonggi-do 13403 (KR)
(72) Inventor: AHN, Jang Hyuk, Seongnam-si Gyeonggi-do 13428 (KR); KIM, Min Hee, Seoul 06017 (KR); JEON, Seon Min, Seoul 05790 (KR); MUNKHJARGAL, Anudari, Seongnam-si Gyeonggi-do 13369 (KR)
(74) Representative: Gille Hrabal Partnerschaftsgesellschaft mbB Patentanwälte
(86) International application number: PCT/KR2022/016412
(87) International publication number: WO 2023/075388

(57) **Abstract**

The present disclosure relates to a precipitant composition that effectively removes substances inhibiting the pure separation of microbial DNA, and a high-concentration, high-purity DNA extraction method applied therewith, and more specifically relates to impurity precipitant composition used for precipitating and removing impurities that inhibit the pure separation of DNA during the process of extracting DNA by dissolving cells in a sample, characterized by consisting of 1.3-2.1 %(w/v) phosphotungstic acid hydrate, 1.2-1.8 %(w/v) zinc acetate dihydrate, 13.0-19.0 %(v/v) acetic acid, with the remainder being water, a DNA extraction method applying said precipitant composition, and a method for amplifying DNA extracted in such a way.

## Description

### Technical Field

The present disclosure relates to a precipitant composition used for extracting microbial DNA from samples, such as emulsified foods, and a DNA extraction method applied therewith, and more specifically, to a precipitant composition that effectively removes substances inhibiting the pure separation of microbial DNA, and a high-concentration, high-purity DNA extraction method applied therewith.

### Background Art

As food safety accidents increase, food safety is being emphasized as a common challenge for humanity and an important global issue. The factors harmful to food safety can be classified into microbiological, chemical, and physical factors, with microbes(microorganisms) constituting the majority. Because the hazards arising from microbes have an immediate and serious impact on human health, diseases caused by microbes are of global concern, and many studies are being conducted for their effective management.

Emulsified foods such as powdered milk, soy milk, chocolate, and ice cream, as well as diary products including milk, fermented milk, and cheese, where water-soluble and lipid-soluble ingredients are well dispersed and mixed, are high-nutritional foods containing proteins, fats, vitamins, and minerals, creating an environment where microbes can easily grow. Therefore, they can be easily contaminated by harmful microbes throughout the entire process of production, transportation, and sale. However, microbes exist in small amounts in food and are mixed with various substances, making it difficult to isolate and detect microbial DNA. Thus, microbial detection methods in food must be able to accurately detect even small amounts of microbes.

The traditional method of microbial detection, the culture medium method, involves cultivating microbes in food under suitable conditions and directly observing the proliferated microbes. However, the culturing method not only requires a lot of labor and time but also has limitations due to frequent false-positive results and low sensitivity. To address these limitations, rapid and simple detection methods are being developed.

The polymerase chain reaction (PCR) technique, based on molecular biology, can detect extremely small amounts of microbes with higher sensitivity than culture methods, and because it amplifies specific parts of extracted DNA, it has high specificity and is widely used in food analysis. Immunological methods using proteins, such as the Enzyme-linked Immunosorbent Assay (ELISA), are also used because they allow for on-site testing, but they tend to have lower sensitivity and reproducibility.

Unfortunately, foods often contain PCR inhibitors such as fats, proteins, and calcium, which can interfere with DNA amplification. It has been reported as a common issue that PCR inhibitors from dairy products need to be removed, and DNA suitable for analysis needs to be extracted, making it crucial to extract DNA with sufficient concentration and purity. Therefore, to successfully perform PCR analysis of microbial DNA extracted from food, it is important to remove PCR inhibitory factors during the DNA extraction process. When extracting microbial DNA from emulsified foods like dairy products, proteins and fats contained in the food act as inhibitors in the pure separation of DNA and PCR reactions, making it essential to remove these substances during the DNA extraction process.

Additionally, microbial detection methods must be able to accurately detect microbes present in trace amounts within food, with the minimum concentration of microbes required for detection referred to as the limit of detection. The factors that affect the limit of detection during PCR are the concentration and purity of DNA. Therefore, if the DNA extraction efficiency is low or there is contamination during the extraction process, PCR may be difficult to perform, making it important to choose an appropriate DNA extraction method for analysis.

Conventional DNA extraction methods involve the use of phenol. The method involves the extraction and separation of DNA using phenol/chloroform or phenol/chloroform/isoamyl alcohol, which is time-consuming and requires caution due to the use of phenol and other reagents harmful to humans. Due to these drawbacks, more straightforward and effective extraction methods are being developed. Currently, column-based DNA extraction methods using a silica membrane or glass fiber that specifically binds to DNA are widely used for their speed and convenience.

For example, commercial kits for extracting microbial DNA from milk or cheese samples, such as the Milk Bacterial DNA Isolation Kit (Norgen Biotek Corporation, Ontario, Canada) or the PowerFood^{™} Microbial DNA Isolation Kit (MoBio Laboratories Inc., Carlsbad, CA, USA) are known. However, while these kits are widely used due to their simple and convenient protocols for DNA extraction, the efficiency of extracting microbial DNA from milk remains low due to inhibitors and impurities contained in the food, such as proteins.

### Disclosure

### Technical Problem

The objective of the present disclosure is to provide a precipitant that effectively removes impurities inhibiting the pure separation of DNA in DNA extraction using a silica membrane column, for high-efficiency extraction of microbial DNA from emulsified foods.

Another objective of the present disclosure is to provide a method for extracting microbial DNA using a silica membrane column, applying the aforementioned precipitant.

### Technical Solution

To achieve the aforementioned objectives, the present disclosure relates to impurity precipitant composition used for precipitating and removing impurities that inhibit the pure separation of DNA during the process of extracting DNA by lysis of cells in a sample, characterized by consisting of 1.3-2.1 %(w/v) phosphotungstic acid hydrate, 1.2-2.8 %(w/v) zinc acetate dihydrate, 13.0-19.0 %(v/v) acetic acid, with the remainder being water, a DNA extraction method applying said precipitant composition, and a method for amplifying DNA extracted in such a way.

### Advantageous Effects of the Invention

As described above, according to the present disclosure, impurities that inhibit the pure separation of DNA can be effectively removed by the precipitant, enabling the extraction of DNA at higher concentrations and purities than with conventional methods.

Additionally, according to the present disclosure, it becomes possible to extract microbial DNA from emulsified foods at high concentrations and purities, thus enabling the efficient and accurate detection of microbes within food, even at low concentrations, compared to conventional methods.

### Brief Description of Drawings

FIG. 1 is an electrophoresis photograph performed after PCR of *Salmonella typhimurium* DNA extracted in the embodiment.
   Lane 1, 7: 1Kb Plus DNA ladder; Lane 2: NTC (Non Template Control); Lanes 3, 4: Control (milk); Lanes 5, 6: Milk samples inoculated with 1/5 *Salmonella typhimurium* in milk.
FIG. 2 is an electrophoresis photograph performed after PCR of *Cronobacter sakazakii* DNA extracted in the embodiment.
   Lane 1, 7: 1Kb Plus DNA ladder; Lane 2: NTC (Non Template Control); Lanes 3, 4: Control (milk); Lanes 5, 6: Milk samples inoculated with 1/5 Cronobacter sakazakii in milk.
FIG. 3 is an electrophoresis photograph performed after PCR of *Cronobacter muytjensii* DNA extracted in the embodiment.
   Lane 1, 7: 1Kb Plus DNA ladder; Lane 2: NTC (Non Template Control); Lanes 3, 4: Control (milk); Lanes 5, 6: Milk samples inoculated with 1/5 *Cronobacter muytjensii* in milk.
FIG. 4 is an electrophoresis photo performed after PCR of *Pseudomonas aeruginosa* DNA extracted in the embodiment.
   Lane 1, 7: 1Kb Plus DNA ladder; Lane 2: NTC (Non Template Control); Lanes 3, 4: Control (milk); Lanes 5, 6: Milk samples inoculated with 1/5 *Pseudomonas aeruginosa* in milk.
FIG. 5 is an electrophoresis photograph performed after PCR of DNA extracted from different bacterial counts of *Salmonella typhimurium* to check the limit of detection in embodiment 5.
   Lane 1: 1Kb Plus DNA ladder; Lane 2: NTC (Non Template Control); Lane 3: Control (milk); Lane 4: Milk sample inoculated with 1/5 *Salmonella typhimurium* in milk; Lanes 5-11: Milk samples inoculated with Salmonella typhimurium from 2×10⁶-2×10° CFU/mL.
FIG. 6 is an electrophoresis photograph performed after PCR of DNA extracted from different bacterial counts of *Cronobacter muytjensii* to check the limit of detection in embodiment 5.
   Lane 1: 1Kb Plus DNA ladder; Lane 2: NTC (Non Template Control); Lane 3: Control (milk); Lane 4: Milk sample inoculated with 1/5 *Cronobacter muytjensii* in milk; Lanes 5-11: Milk samples inoculated with *Cronobacter muytjensii* from 2×10⁶-2×10° CFU/mL.

### Best Mode for Invention

The present disclosure will now be described in more detail with reference to the accompanying drawings and embodiments. However, these drawings and embodiments are merely illustrative of the concepts and scope of the present disclosure and are not intended to limit or modify its technical scope. It will be apparent to those skilled in the art that various modifications and variations can be made within the scope of the technical concepts of the present disclosure based on these examples.

The inventors of the present disclosure have completed this invention upon discovering that in the extraction of microbial DNA from food, the presence of PCR inhibitors such as proteins and fats, or the mixture of various substances contained in the food results in low recovery rates of microbial DNA, showing inefficiency in conventional methods or kits, and that removing impurities which inhibit the pure separation of DNA during the extraction phase can increase the extraction rate of microbial DNA. That is, the inventors developed a precipitant composition to remove impurities that obstruct DNA extraction, derived a method for efficiently extracting microbial DNA from dairy products using it, and completed the invention by accurately determining the presence of microbial detection through PCR of the extracted DNA.

During the completion process of the present disclosure, a complex and multistep process of preparing precipitant compositions with various components and component ratios, and applying these to conventional methods of DNA separation from dairy products (for example, methods using the Milk Bacterial DNA Isolation Kit from Norgen Biotek Corporation, Ontario, Canada) to determine the optimal precipitant composition was initially conducted. However, as the process of determining the precipitant composition includes the unique know-how of the inventors, the explanation will be based on the precipitant composition confirmed through this process. That is, the explanation will proceed in the order of ⓐ manufacturing the precipitant composition according to the present disclosure, ⓑ verifying the difference between applying and not applying the precipitant composition to known methods of DNA separation from dairy products, and ⓒ the improved DNA separation method that includes the precipitating agent composition, and the composition of other buffers used in it (The sequence of embodiments follows accordingly).

### (1) Invention related to the precipitant composition

As previously mentioned, the present disclosure relates to impurity precipitant composition used for precipitating and removing impurities that inhibit the pure separation of DNA during the process of extracting DNA by lysis of cells in a sample, characterized by consisting of 1.3-2.1 %(w/v) phosphotungstic acid hydrate, 1.2-1.8 %(w/v) zinc acetate dihydrate, 13.0-19.0 %(v/v) acetic acid, with the remainder being water. That is, acetic acid is added to a solution in which phosphotungstic acid hydrate and zinc acetate dihydrate are dissolved, and water is added to make up the rest, so that the final volume of the composition is 100%. It is obvious that if the purity of the reagents is low, the content of the reagents can be adjusted to reflect the purity. For example, as in the embodiment, when using phosphotungstic acid hydrate with a purity of 65-70%, the actual amount of phosphotungstic acid hydrate used can be adjusted to 2.0-3.0 %(w/v).

The composition of the precipitating agent composition was determined by utilizing the widely used statistical technique of Response Surface Methodology (RSM), which allows for statistical optimization from experimental design to result analysis, to establish the optimal range of precipitation conditions. The optimal ratio that yields a good precipitation rate was efficiently determined by analyzing the results of the precipitation effect according to the composition ratio of each precipitant using statistical techniques.

### (2) DNA extraction method applying the precipitant composition

Furthermore, the present disclosure provides a method for extracting microbial DNA from samples, such as emulsified foods, with high efficiency using the precipitant composition, specifically comprising the steps of: (A) adding a cell lysis buffer to the sample solution to lyse the cells; (B) adding the precipitant composition prepared in (1) to the lysis sample solution to precipitate impurities; (C) subsequently, transferring the supernatant, excluding the precipitate, from the sample solution to a silica membrane column to immobilize the DNA; (D) then using a washing buffer to clean the column; (E) finally, applying a DNA elution buffer to the column to elute the DNA.

In the DNA extraction method according to the present disclosure, it comprises three types of buffers for DNA extraction, namely, the cell lysis buffer, the washing buffer, and the DNA elution buffer, and it is preferable that their compositions are as follows.
(a) Cell lysis buffer; 0.4-0.6 %(w/v) N-laurylsarcosine, 25-35mM Tris-HCl (pH 8.0±0.2), 13-17mM EDTA (Ethylenediaminetetraacetic acid, pH 8.0±0.2), 2.5-3.5M Guanidine thiocyanate
ⓑ Washing buffer; 9-11mM Tris-HCl (Tris hydrochloride, pH 8.0±0.2) and 75-85% Ethanol
ⓒ Elution buffer; 9-11mM Tris-HCl (Tris hydrochloride, pH 8.0±0.2) and 0.9-1.1mM EDTA (Ethylenediaminetetraacetic acid, pH 8.0±0.2)

The DNA extraction method of the present disclosure includes an additional step of impurity removal through the precipitant in the basic column-based DNA extraction process using the DNA extraction buffers. During the extraction process using the DNA extraction buffers and the precipitant, the precipitant effectively removes impurities other than DNA, allowing for the extraction of high-purity DNA. As can be seen in the following embodiment, the use of the precipitant composition improved the efficiency of DNA extraction compared to conventional methods, and the purity of the extracted DNA (A260/280) was significantly enhanced.

Additionally, when comparing the efficiency of DNA extraction using the extraction method of the present disclosure and an existing commercial kit for extracting microbial DNA from milk, it was confirmed that using the method of the present disclosure allowed for DNA extraction with higher efficiency from the same sample and quantity.

Using the extraction method of the present disclosure to determine the limit of DNA detection according to bacterial count, it was detected down to 2×10¹ CFU/mL, meaning that microbes present at a low concentration of 2×10¹ CFU (Colony Forming Unit) per 1 mL of sample (milk) can be detected.

Therefore, the precipitant and DNA extraction method according to the present disclosure can detect microbial DNA present at low concentrations in emulsified foods, making it usefully applicable to the analysis of microbial detection within food.

### [Embodiments]

### 1. Microbial Cultivation and Count Measurement

As shown in Table 1 below, four types of microbes were each cultivated in their respective media at 36°C for about 17 hours, and their counts were measured.

**[Table 1]**

| **Strain** | **(ATCC)** | **Medium** |
|---|---|---|
| *Salmonella typhimurium* | 14028 | Salmonella enrichment base (3M Healthcare, USA) |
| *Cronobacter sakazakii* | 29544 | Nutrient broth (EMD Millipore Coorporation, Germany) |
| *Cronobacter muytjensii* | 51329 | Tryptic Soy Broth (Becton, Dickinson and Company, USA) |
| *Pseudomonas aeruginosa* | 27853 | |

Using sterilized milk, the culture solution was diluted in the range of 10⁻¹-10⁻⁶, and then 100 µL of the culture solution diluted from 10⁻⁴-10⁻⁶ was spread on plate count agar (PCA, BD Difco) medium and incubated at 36°C for 24 hours before the bacterial count was measured.

### 2. Precipitant Composition According to the Present Disclosure

Precipitant composition was prepared, consisting of 2.0-3.0 %(w/v) phosphotungstic acid hydrate (purity 65.0-70.0%), 1.2-1.8 %(w/v) zinc acetate dihydrate, 13.0-19.0 %(v/v) acetic acid, with the remainder being water.

### 3. Verification of the Effects of the Present Disclosure: Application Example 1 and Comparative Example 1

First, the amount and purity of DNA extracted using an existing commercial kit, the Milk Bacterial DNA Isolation Kit (Norgen Biotek Corp.), according to the manufacturer's instructions (Comparative Example 1), were compared with those extracted by adding a step using the precipitant composition according to the present disclosure to the basic kit and instructions (Application Example 1).

The count of the microbes cultured in 1 was adjusted to 8 log CFU/mL, and this culture was artificially inoculated into sterilized milk and diluted to be used as a sample (hereinafter the same).

### (1) Comparative Example 1

DNA was extracted using the existing commercial kit, Milk Bacterial DNA Isolation Kit, as follows:
① The sample was centrifuged at 14,000 rpm for 3 minutes, the supernatant was removed, then 400 µL of Buffer SK was added and mixed well.
② After cell lysis, 200 µL of 96-100% ethanol was added, mixed, then the solution was transferred to a column and centrifuged at 14,000 rpm for 2 minutes.
(3) The solution that has passed through the column was removed, 500 µL of Buffer SK was added, and it was centrifuged at 14,000 rpm for 2 minutes.
④ The solution that has passed through the column was again removed, 500 µL of Wash Solution A was added, and it was centrifuged at 14,000 rpm for 1 minute. This process was repeated twice, and then the column was spun at 14,000 rpm for 2 minutes to dry.
(5) The column was transferred to a new tube, 100 µL of Elution buffer B was added, and it was centrifuged at 2,000 rpm for 2 minutes to elute the final DNA.

### (2) Application Example 1

Apart from adding the step of treating with the precipitant composition prepared in 2, the process was carried out in the same manner as Comparative Example 1 in (1). Specifically, in Comparative Example 1 of (1), 20 µL of the precipitant was added to the solution in step CD, mixed, and then centrifuged at 13,000 rpm for 5 minutes. After transferring the supernatant to a new tube, the process was carried out in the same manner as from the step of adding and mixing 200 µL of ethanol in Comparative Example 1 step ②.

### (3) Result Review

The concentration and purity of the DNA extracted in this way were measured and are presented in Table 2 below.

The concentration and purity of DNA were measured using a spectrophotometer (NP80 NanoPhotometer UV/Vis Spectrophotometers (Implen)) by adjusting the blank with DNA elution buffer and using 2 µL of the extracted DNA for the measurement.

DNA purity was determined by measuring the absorbance at 260nm and 280nm and checking the A260/280 ratio (the same hereafter). An A260/280 value between 1.8 and 2.1 is considered to indicate pure nucleic acid.

**[Table 2]**

| **Strain** | | **Concentration (ng/µL)** | **Concentration Ratio(%)** | **A260/280** |
|---|---|---|---|---|
| *S. typhimurium* | Comparative Example 1 | 2.35 | 100.0 | 1.05 |
| | Application Example 1 | 3.20 | 136.2 | 1.94 |
| *C. sakazakii* | Comparative Example 1 | 2.10 | 100.0 | 2.56 |
| | Application Example 1 | 3.38 | 161.0 | 1.89 |
| *C. muytjensii* | Comparative Example 1 | 6.70 | 100.0 | 1.51 |
| | Application Example 1 | 8.05 | 120.1 | 1.92 |
| *P. aeruginosa* | Comparative Example 1 | 5.07 | 100.0 | 1.75 |
| | Application Example 1 | 5.53 | 109.1 | 1.83 |

As shown in the table, according to Application Example 1 of the present disclosure, the concentration (amount) of DNA extracted was increased for all compared to that extracted using the existing commercial kit (Comparative Example 1). In terms of DNA purity (A260/280), all values in Application Example 1 were above 1.8, corresponding to the range of 1.8-2.1, which indicates the best purity, unlike the results of Comparative Example 1.

Thus, the extraction method according to the present disclosure (Application Example 1) results in the extraction of DNA at concentrations (amounts) that are nearly identical to or slightly higher than those achieved using the existing commercial kit (Comparative Example 1), however, in terms of DNA purity, the method of the present disclosure allows for the extraction of DNA at high purity, confirming that this extraction method is more efficient in extracting high-concentration, high-purity DNA.

### 4. Adaptability to Other Protocols

The above analysis and review of the extraction method of the present disclosure were somewhat limited by adopting the procedures of the conventional technology (commercialized kit), except for the application of the precipitant, for comparison with the conventional technology. Therefore, beyond the protocols of the conventional technology, other methods were established to verify whether the present disclosure can effectively extract a large amount of DNA at high purity.

To achieve this, buffers for DNA extraction were prepared to replace those of the conventional commercialized kit (whose compositions are not disclosed), and experiments were conducted using a specific protocol with modified extraction process.

### (1) Preparation of DNA Extraction Buffers

Three types of buffers for DNA extraction were prepared: cell lysis buffer, washing buffer, and DNA elution buffer.

The cell lysis buffer was prepared with a composition of 0.4-0.6 %(w/v) N-laurylsarcosine, 25-35mM Tris-HCl (pH 8.0±0.2), 13-17mM EDTA (Ethylenediaminetetraacetic acid, pH 8.0±0.2), and 2.5-3.5M Guanidine thiocyanate.

The washing buffer was prepared with a composition of 9-11mM Tris-HCl (Tris hydrochloride, pH 8.0±0.2) and 75-85% Ethanol.

The DNA elution buffer was prepared with a composition of 9-11mM Tris-HCl (Tris hydrochloride, pH 8.0±0.2) and 0.9-1.1mM EDTA (Ethylenediaminetetraacetic acid, pH 8.0±0.2).

### (2) Modified Protocol

While the details are omitted, through repeated extraction experiments using the buffers described in (1) and the precipitant of the present disclosure, a partially optimized modified protocol was established as follows.

### <Comparative Example 2>

① The sample was centrifuged at 13,000 rpm for 5 minutes, the supernatant was removed, then 500 µL of lysis buffer was added and dissolved at 65° C for 10 minutes.
② The solution from step ① was transferred to a silica membrane column and centrifuged at 13,000 rpm for 1 minute.
③ The solution that has passed through the column was removed, 500 µL of washing buffer was added, and it was centrifuged at 13,000 rpm for 1 minute. This process was repeated twice, and then the column was spun at 13,000 rpm for 3 minutes to dry.
④ The column was transferred to a new tube, 100 µL of elution buffer was added, it was left at room temperature for 5 minutes, then centrifuged at 13,000 rpm for 1 minute to elute the final DNA.

### <Application Example 2>

The process involving the precipitant composition was added to Comparative Example 2 from above. Specifically, in Comparative Example 2, 20 µL of the precipitant was added to the solution from step CD, mixed, and then centrifuged at 13,000 rpm for 5 minutes. After transferring the supernatant to the silica membrane column as in step ② of Comparative Example 2, the procedure was carried out in the same manner.

### (3) Result Review

The concentration and purity of the DNA extracted in this manner were measured and are presented in Table 3 below.

**[Table 3]**

| **Strain** | | **Concentration (ng/µL)** | **Concentration Ratio(%)** | **A260/280** |
|---|---|---|---|---|
| *S. typhimurium* | Comparative Example 2 | 4.80 | 100 | 1.70 |
| | Application Example 2 | 8.08 | 168.3 | 1.88 |
| *C. sakazakii* | Comparative Example 2 | 4.75 | 100 | 1.85 |
| | Application Example 2 | 7.77 | 163.6 | 1.83 |
| *C. muytjensii* | Comparative Example 2 | 3.05 | 100.0 | 1.07 |
| | Application Example 2 | 12.00 | 393.4 | 1.82 |
| *P. aeruginosa* | Comparative Example 2 | 6.80 | 100.0 | 1.41 |
| | Application Example 2 | 18.27 | 268.7 | 1.83 |

As shown in the table, even with the alternative protocol, Application Example 2, which uses the precipitant composition of the present disclosure, resulted in a roughly 150-300% increase in the concentration (amount) of DNA extracted from all microbes compared to that extracted without the precipitant (Comparative Example 2), additionally, the purity of DNA (A260/280) was below 2.1 for all, falling within the range of 1.8-2.1, which indicates the best purity (A260/280).

### 5. Analysis of the Characteristics of the Present Disclosure

The experiments confirmed that the method of processing the precipitant during the DNA extraction process according to the present disclosure (Application Example 1) significantly outperforms the extraction efficiency compared to the method using the existing widely utilized commercial kits (Comparative Example 1). Furthermore, the method using the precipitant (Application Example 2) also showed equal or higher extraction efficiency in the modified extraction method using new DNA extraction buffers (Comparative Example 2).

The DNA extracted in this manner was analyzed to determine whether it could be used as a template for subsequent processes, namely amplification, and to analyze the limit of detection of the extraction method according to the present disclosure.

### (1) Verification of PCR Analysis Using Extracted DNA

To verify whether DNA extracted using the established extraction method from the above experiment is suitable for PCR analysis without any issues, PCR was conducted using DNA obtained by Application Example 2.

PCR was performed using a T100 Thermal Cycler (Biorad) device. Primer sequences were referenced from the papers by Fratamico, 2003 (Salmonella typhimurium), Jang et al., 2020 (Cronobacter), and Xu et al., 2004 (Pseudomonas aeruginosa).

The PCR mixture consisted of 10 µL of DreamTaq PCR Master mix (Thermo Fisher Scientific), 1 µL of 10 µM forward and reverse primers each, 1 µL of DNA, and 7 µL of sterile distilled water. The PCR reaction conditions were one cycle at 95°C for 3 minutes; a total of 35 cycles of 1 cycle at 95°C for 30 seconds, 55°C for 30 seconds, and 72°C for 1 minute; followed by one cycle at 72°C for 5 minutes.

Electrophoresis was performed using the E-gel power snap electrophoresis system (Thermo Fisher Scientific), and analysis was conducted using a 1Kb Plus DNA ladder (Thermo Fisher Scientific) and 2% E-Gel EX agarose gel (Invitrogen by Thermo Fisher Scientific) .

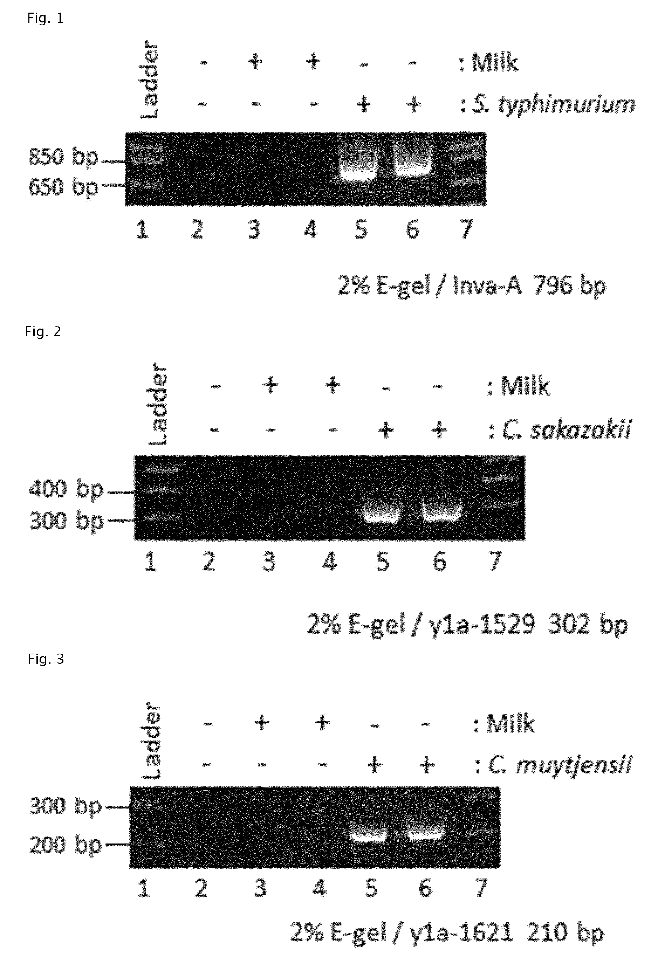

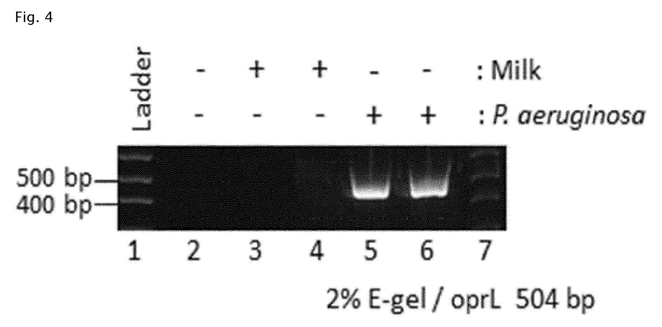

The results of the electrophoresis after PCR are illustrated in FIGs. 1 to 4. As shown in the figures, *S*. *typhimurium* was amplified at 796 bp (invA gene) (FIG. 1), *C*. *sakazakii* at 302 bp (yla fimbriae gene), *C. muytjensii* at 210 bp (γ1a fimbriae gene) were confirmed (FIG. 2, 3), and *P. aeruginosa* was amplified at 504 bp (oprL (outer membrane lipoprotein) gene) (FIG. 4). Thus, the DNA extracted for all four types of microbes using Application Example 2 was amplified from each target gene, confirming that the DNA extracted according to the present disclosure is suitable for PCR analysis without any issues.

### (2) Verification of DNA Extraction Limit of Detection According to Bacterial Count

The microbial DNA extraction method according to the present disclosure is crucial in terms of DNA extraction efficiency and the limit of detection because it does not involve a culturing process. Therefore, the final limit of detection was verified according to bacterial count by performing DNA extraction and PCR using the extraction method of Application Example 2 according to the present disclosure.

To verify the limit of detection of the microbial DNA extraction method according to the present disclosure, cultures of *S. typhimurium* and *C*. *muytjensii* were each serially diluted with sterilized milk (from 2×10⁶ to 2×10⁰ CFU/mL), and 1 mL from each dilution was collected to extract DNA using the method as described in Application Example 2, PCR analysis was then performed according to the method described in (1).

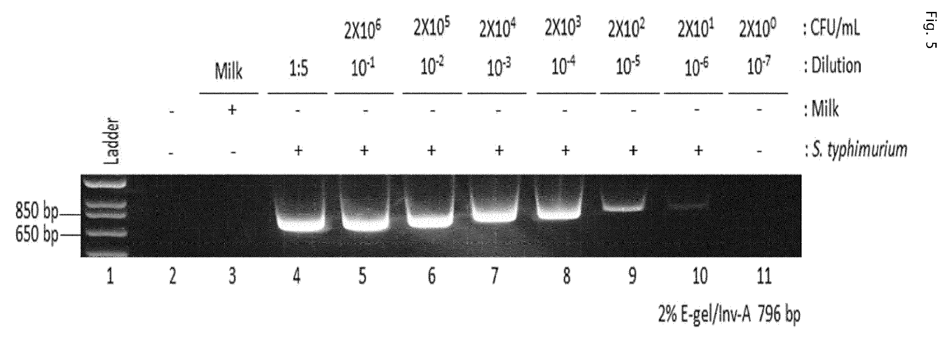

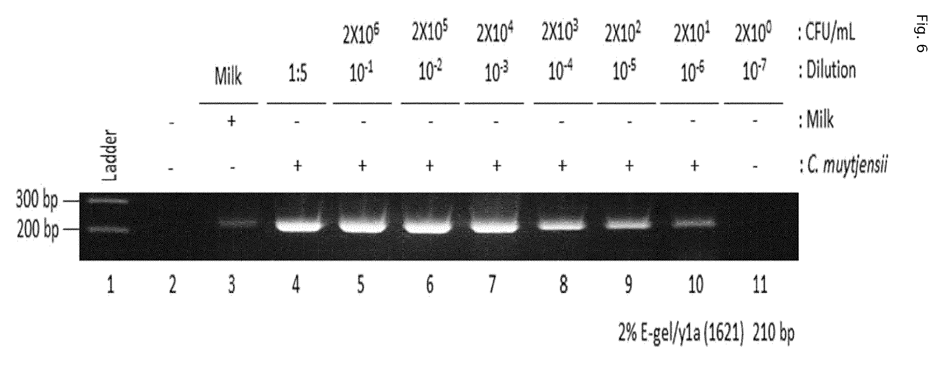

The results of the DNA extraction and PCR analysis are presented in FIG. 5, 6, and Table 4 below. As can be seen in the figures and table, the limit of detection for the microbial DNA extraction method according to the present disclosure was found to be 2×10¹ CFU/mL for both *S. typhimurium* and *C*. *muytjensii,* indicating that microbes present at a low concentration of 2×10¹ CFU (Colony Forming Unit) per 1 mL of milk can be detected.

**[Table 4]**

| **Strain** | **PCR (CFU/mL)** | | | | | | |
|---|---|---|---|---|---|---|---|
| | 2X10⁶ | 2X10⁵ | 2X10⁴ | 2X10³ | 2X10² | 2X10¹ | 2X10⁰ |
| *S. typhimurium* | + | + | + | + | + | + | - |
| *C. muytjensii* | + | + | + | + | + | + | - |

| | | | | | | | |
|---|---|---|---|---|---|---|---|
| +: Detected, -: Not detected | | | | | | | |

Therefore, the extraction method according to the present disclosure (Application Example 1, 2) results in the extraction of high-purity DNA that is equivalent to or better than that extracted using the existing commercial kit (Comparative Example 1) or a modified extraction method (Comparative Example 2), and also achieves extraction at a significantly higher DNA concentration (amount). This demonstrates that the precipitant composition and the DNA extraction method according to the present disclosure show superior effects compared to conventional technology, and further optimization could lead to even better results.

## Claims

1. An impurity precipitant composition used for precipitating and removing impurities that inhibit the pure separation of DNA during the process of extracting DNA by lysis of cells in a sample comprising:
1.3-2.1 %(w/v) phosphotungstic acid hydrate, 1.2-1.8 %(w/v) zinc acetate dihydrate, 13.0-19.0 %(v/v) acetic acid, with the remainder being water.

2. A method for extracting DNA comprising the steps of:
(A) adding a cell lysis buffer to the sample solution to lyse the cells;
(B) adding the precipitant composition according to claim 1 to the lysis sample solution to precipitate impurities;
(C) subsequently, transferring the supernatant, excluding the precipitate, from the sample solution to a silica membrane column to immobilize the DNA;
(D) then using a washing buffer to clean the column;
(E) finally, applying a DNA elution buffer to the column to elute the DNA.

3. The method for extracting DNA of claim 2, wherein the lysis buffer is composed of 0.4-0.6 %(w/v) N-laurylsarcosine, 25-35mM Tris-HCl (pH 8.0±0.2), 13-17mM EDTA (Ethylenediaminetetraacetic acid, pH 8.0±0.2), and 2.5-3.5M Guanidine thiocyanate,
the washing buffer is composed of 9-11mM Tris-HCl (Tris hydrochloride, pH 8.0±0.2) and 75-85% Ethanol,
the DNA elution buffer is composed of 9-11mM Tris-HCl (Tris hydrochloride, pH 8.0±0.2) and 0.9-1.1mM EDTA (Ethylenediaminetetraacetic acid, pH 8.0±0.2).

4. The method for extracting DNA of claim 2 or 3, wherein the sample is an emulsified food or derived from emulsified food.

5. A method for amplifying DNA comprising the use of DNA extracted according to the method of claims 2 or 3 as a template.
